# EUROPÄISCHE PATENTSCHRIFT

(11) **EP 2 467 682 B1**
(45) Veröffentlichungstag und Bekanntmachung des Hinweises auf die Patenterteilung: **31.07.2013**
(21) Anmeldenummer: 10798265.4
(22) Anmeldetag: 17.12.2010
(51) Int. Cl.: G01F 23/24

(54) **MESSANORDNUNG FÜR EINE FLÜSSIGKEIT, INSBESONDERE ZUR VERWENDUNG IN EINER MILCHANNAHMEANORDNUNG, UND VERFAHREN ZUM BETRIEB EINER MILCHANNAHMEANORDNUNG**
MEASUREMENT DEVICE FOR A LIQUID, IN PARTICULAR FOR USE IN A MILK COLLECTION ARRANGEMENT, AND METHOD FOR OPERATING A MILK COLLECTION ARRANGEMENT
DISPOSITIF DE MESURE POUR UN LIQUIDE, DESTINE À ÊTRE UTILISE EN PARTICULIER DANS UN SYSTEME COLLECTEUR DE LAIT, ET PROCEDE PERMETTANT DE FAIRE FONCTIONNER UN SYSTEME COLLECTEUR DE LAIT

(30) Priorität: 18.12.2009 DE 102009058838
(43) Veröffentlichungstag der Anmeldung: 27.06.2012
(73) Patentinhaber: Bartec Benke GmbH, 97980 Bad Mergentheim (DE)
(72) Erfinder: BÖHM, Alfred, 94234 Viechtach (DE)
(74) Vertreter: Kohl, Karl-Heinz
(86) Internationale Anmeldenummer: PCT/EP2010/007760
(87) Internationale Veröffentlichungsnummer: WO 2011/072874

(56) Entgegenhaltungen:
- WO-A1-2007/006311
- DE-A1-102006 014 207

## Beschreibung

Die Erfindung betrifft eine Messanordnung für eine Flüssigkeit, insbesondere zur Verwendung in einer Flüssigkeitsannahmeanordnung, bevorzugt Milchannahmeanordnung, gemäß dem Oberbegriff des Anspruchs 1. Eine solche Messanordnung ist ausgebildet mit einem Messrohr zum Durchleiten der Flüssigkeit und zwei länglichen Elektroden, die zumindest bereichsweise einander gegenüberliegend im Messrohr angeordnet sind.

Die Erfindung betrifft ferner die Verwendung einer solchen Messanordnung in einer Flüssigkeitsannahmeanordnung sowie ein Verfahren zum Betrieb einer Flüssigkeitsannahmeanordnung.

In der Milchwirtschaft werden täglich große Mengen Milch von den einzelnen Erzeugern (Bauern, Farmern) zu den Verarbeitern (Molkereien, Lebensmittelfabriken) befördert. Dieser Transport geschieht fast ausschließlich durch Milchsammelwagen. Solche Milchsammelwagen weisen einen oder mehrere Sammeltanks auf, sowie eine Milchannahmeanordnung, die zum Überführen der Milch in den Tank und zum Bestimmen der überführten Flüssigkeitsmenge dient.

Ein Beispiel einer derartigen Milchannahmeanordnung ist aus der WO 2008/141664 A1 bekannt. Zur Bestimmung der geförderten Menge weist die bekannte Milchannahmeanordnung eine Messstrecke mit einer Durchflussmesseinrichtung und einer Gasanteilsmesseinrichtung auf, wobei die Gasanteilsmesseinrichtung auch als Füllgradmesseinrichtung bezeichnet werden kann. Die nach der WO 2008/141664 A1 vorgesehene Gasanteilsmesseinrichtung (Füllgradmesseinrichtung) ermöglicht es, Gasanteile in der Flüssigkeit zu tolerieren und diese messtechnisch zu berücksichtigen. Im Hinblick auf große Gaseinschläge, die häufig zu Beginn und zum Ende der Übergabe auftreten, ist nach der WO 2008/141664 A1 ferner ein Speicherbehälter vorgesehen, der in einem Seitenarm der Förderleitung angeordnet ist, und der eine Beruhigung und Entgasung von stark gasdurchsetzten Flüssigkeitsmengen ermöglicht.

Ausführungsformen von Füllgradmesseinrichtungen beziehungsweise Gasanteilsmesseinrichtungen zur Verwendung in einer Milchannahmeanordnung gehen beispielsweise aus der nächstkommenden WO 2006/063631 A1 hervor. Gemäß der WO 2006/063631 A1 wird der Füllgrad/Gasanteil durch eine Leitfähigkeitsmessung ermittelt. Hierzu sind in einem Messrohr Elektroden vorgesehen, die zur Vermeidung von Messfehlern von isolierenden Wandbereichen umgeben sind.

Milchannahmeanordnungen wie beispielsweise in der WO 2008/141664 A1 beschrieben weisen eine Annahmepumpe auf. Diese dient dazu, einen Unterdruck zu erzeugen, um die Milch anzusaugen und sie dann mit Überdruck in den Sammeltank zu befördern. Dabei können bei der Milchannahme unterschiedliche Annahmebedingungen angetroffen werden, nämlich eine verglichen mit der Pumpe und/oder dem Sammeltank des Milchsammelwagens erhöhte Anordnung des Abgabetanks des Erzeugers, eine ebenerdige Anordnung des Abgabetanks oder eine unterirdische Anordnung des Abgabetanks.

Bei der Übergabe fließt die Milch bei gegebenen Annahmebedingungen umso schneller, je größer der Unterdruck am Pumpeneingang, also der Saugseite ist. Obwohl es im Hinblick auf den Zeitfaktor und die Wirtschaftlichkeit erstrebenswert wäre, eine möglichst große Pumpleistung zu realisieren, kann die Pumpleistung in der Praxis durch das Auftreten von Kavitation beschränkt sein. Kavitation, das heißt die lokale Verdampfung infolge örtlichen Unterdrucks, ist ein bekanntes Phänomen und beispielsweise in der DE 2 452 641 A beschrieben. Bei der Förderung von Milch ist Ka-, vitation in der Regel bereits deshalb zu vermeiden, da sie das Potenzial hat, die Milch zu schädigen. Daher ist für die Pumpe ein Betriebszustand anzustreben, bei dem die Pumpleistung so hoch ist, dass Kavitation gerade noch vermieden wird. Im Hinblick auf die Kavitationsproblematik lehrt die WO 2008/141664 A1 demgemäß, den Saugdruck der Pumpe auf einen Wert zu regeln, bei dem Kavitation der Flüssigkeit unterbunden ist.

Zum Nachweis von Kavitation in verschiedenen Situationen sind Kavitationssensoren bekannt. So ist gemäß der DE 24 52 641 A ein Kavitationssensor auf akustischer Basis für ein Wasserfahrzeug bekannt. Die DE 699 37 747 T2 enthält den Gedanken, akustische Emissionen, eine optische Streuung, eine Hochgeschwindigkeitsfotografie, eine mechanische Beschädigung oder Sonochemikalien zur Erfassung von Kavitation zu verwenden. Gemäß der DE 691 30 592 T2 wird ein Beschleunigungsmesser oder ein akustischer Wandler als Kavitationssensor eingesetzt. Gemäß DE 197 25 012 C1 werden die Ausbreitungseigenschaften von Rayleigh-Wellen herangezogen, um Kavitation nachzuweisen. Die DE 197 15 480 A1 lehrt die Verwendung eines akustischen Kavitationssensors. Die DE 100 45 847 A1 lehrt einen Kavitationssensor mit einem piezoelektrischen Element.

Aus der WO 2007/006311 A1 ist eine Messanordnung zum Bestimmen der Durchflussrate von Milch in einer Mischung aus Milch und Luft mit einer Einrichtung zum Abtrennen der Luft bekannt. Ferner ist eine offene Messkammer beschrieben in welcher Messelektroden angeordnet sind. Es wird der Füllgrad der Messkammer mittels Messelektroden ermittelt und in einen Durchflusswert umgerechnet.

Aus der DE 10 2006 014 207 A1 ist eine Milchsammeleinrichtung und ein Verfahren zum Betreiben von einer Milchsammeleinrichtung bekannt. Hierbei weist die Milchsammeleinrichtung zwei Elektroden auf, die in Längsrichtung der Messvorrichtung im Wesentlichen parallel zueinander verlaufen.

Durch den Milchtransport entstehen Kosten, die es zu minimieren gilt. Hierzu bedient man sich zum einen der Tourenoptimierung. Zum anderen wird versucht, die Annahmeleistung der Milchsammelwagen möglichst hoch zu wählen, wobei beachtet werden muss, dass die Milch nicht beschädigt werden darf. Schließlich kann eine Kostenreduktion durch Bereitstellung einer besonders kostengünstigen und wirtschaftlichen Annahmeanordnung erreicht werden.

**Aufgabe** der Erfindung ist es, eine Messanordnung, insbesondere für eine Milchannahmeanordnung, und Verfahren zum Betrieb solcher Anordnungen anzugeben, die eine besonders große Einsatzvielfalt und eine besonders hohe Wirtschaftlichkeit gewährleisten.

Die Aufgabe wird erfindungsgemäß durch eine Messanordnung mit den Merkmalen des Anspruchs 1, der Verwendung einer solchen Messanordnung gemäß Anspruch 11 und einem Verfahren zum Betrieb einer Milchannahmeanordnung mit den Merkmalen des Anspruchs 12 gelöst. Bevorzugte Ausführungsbeispiele sind in den abhängigen Ansprüchen angegeben.

Die erfindungsgemäße Messanordnung ist dadurch gekennzeichnet, dass die Messanordnung zur Kavitationsmessung mit der Flüssigkeit befüllbar ist, wobei das Auftreten von Kavitation durch eine Änderung der Leitfähigkeit zwischen den beiden Elektrode aufgrund von Kavitationsblosen erkannt wird und die Messanordnung in einem Förderbetrieb von der Flüssigkeit, In senkrechter Richtung durchströmt wird.

Gemäß einem Grundgedanken der Erfindung wird eine Messanordnung bereitgestellt, die eine Leitfähigkeitsmessung der durchfließenden Flüssigkeit erlaubt. Aufgrund ihrer besonderen Elektrodenanordnung kann dabei ein und dieselbe Messanordnung für mindestens drei verschiedene Funktionen eingesetzt werden. So kann durch Leitfähigkeitsmessung zwischen den Elektroden zum einen der Füllgrad, das heißt der Flüssigkeits- beziehungsweise Gasanteil in der Flüssigkeit bestimmt werden. Der hierbei bestimmte Füllgrad kann dazu verwendet werden, die Messergebnisse einer Durchflussmesseinrichtung im Hinblick auf Gasanteile zu korrigieren. Darüber hinaus fungiert die Messanordnung als Kavitationssensor. Denn tritt in der Flüssigkeit Kavitation auf, so kommt es aufgrund der Kavitationsblasen zu einer Änderung der Leitfähigkeit zwischen den beiden Elektroden. Wird dieser Effekt nachgewiesen, so kann die Fördergeschwindigkeit, der Saugdruck und/oder die Leistung einer Förderpumpe entsprechend verringert werden, so dass die Kavitation wieder unterbunden ist.

Schließlich kann die erfindungsgemäße Messanordnung aufgrund der Elektrodenanordnung aber auch als Niveausensor verwendet werden, um beispielsweise nach Abschluss der Übergabe das Füllniveau in einem Förderleitungsverlauf zu bestimmen. Denn aufgrund der erfindungsgemäßen Anordnung von Elektroden und Messrohr stehen die Elektroden zumindest annähernd senkrecht und somit rechtwinklig zur Flüssigkeitsoberfläche, die am Abschluss der Übergabe das Füllniveau definiert. Aufgrund dieser senkrechten Anordnung hat eine Füllniveauänderung eine deutliche Änderung des Leitfähigkeitswertes zwischen den Elektroden zur Folge, so dass durch Leitfähigkeitsmessung ein besonders repräsentatives Signal für das Füllniveau erhalten werden kann. Der gleichzeitig vorgesehene erfindungsgemäß senkrechte Rohrverlauf hat, insbesondere bei konstantem Rohrquerschnitt im Bereich der Elektroden, dabei überdies zur Folge, dass eine mathematisch besonders einfache Beziehung zwischen dem gemessenen Leitfähigkeitswert und dem Füllniveau besteht, was die Auswertung stark vereinfacht.

Unter der Rohrachse kann insbesondere die Längs- und/oder Symmetrieachse des Messrohres verstanden werden. Unter dem erfindungsgemäßen Verlauf der Elektroden längs der Rohrachse kann insbesondere verstanden werden, dass die Längsachsen der Elektroden (das heißt jene Achsen der Elektroden, welche der Richtung der größten Elektrodenausdehnung entsprechen), parallel zur Rohrachse verlaufen.

Auch kann dieser Gedanken beinhalten, dass die Elektrodenausdehnung in Richtung der Rohrachse größer ist als quer zur Rohrachse, insbesondere um zumindest den Faktor 2 oder 5 größer. Die Erfindung beinhaltet somit, dass die Elektroden in Strömungsrichtung der Flüssigkeit verlaufen, welche im Bereich der Elektroden die Senkrechte ist.

Die Elektroden können beispielsweise zumindest eine rechteckige Kontaktoberfläche für die Flüssigkeit aufweisen, wobei dann erfindungsgemäß die längere Rechteckseite parallel zur Rohrachse des Messrohres verläuft. Die Elektroden können aber beispielsweise auch stabförmig ausgebildet sein, wobei dann die Längs- oder Symmetrieachse des Stabes parallel zur Rohrachse verlaufen kann.

Das erfindungsgemäße Messrohr weist zweckmäßigerweise eine Zulauföffnung sowie eine Auslauföffnung auf, so dass die zu charakterisierende Flüssigkeit durch das Messrohr hindurchgeleitet werden kann. Im Bereich dieser Öffnungen sind geeigneterweise Anschlüsse, insbesondere Flanschelemente vorgesehen, so dass das Messrohr in einen Förderleitungsverlauf einer Annahmeanordnung eingebaut werden kann. Im Bereich der Elektroden kann das Messrohr beispielsweise einen runden, insbesondere kreisrunden, einen rechteckigen oder einen quadratischen Innenquerschnitt aufweisen. Sofern im Bereich der Elektroden ein eckiger Querschnitt vorgesehen ist, ist es vorteilhaft, dass das Messrohr im Bereich der Öffnungen und/oder Anschlüsse einen runden Innenquerschnitt aufweist, so dass ein strömungstechnisch günstiger Übergang zum Leitungssystem gegeben ist. Erfindungsgemäß weist das Messrohr eine zum Beispiel hohlzylindrische Rohrwand auf. Diese Rohrwand kann eine Messkammer im Inneren des Rohres definieren, die von der Flüssigkeit durchströmt wird, und mit der die Elektroden in Kontakt stehen.

Besonders vorteilhaft ist es, dass die Rohrwand zumindest an der Rohrinnenseite ein elektrisches Isolatormaterial aufweist. Gemäß dieser Ausführungsform ist die Rohrwand an ihrer Kontaktfläche mit der zu messenden Flüssigkeit nichtleitend ausgebildet, was im Hinblick auf die Messgenauigkeit vorteilhaft sein kann. Die Rohrwand kann auch zur Gänze aus dem Isolatormaterial bestehen. Bei dem Isolatormaterial kann es sich insbesondere um ein Kunststoffmaterial handeln. Die Elektroden sind geeigneterweise für einen elektrischen Kontakt mit der Flüssigkeit ausgebildet, das heißt sie sind im Inneren des Messrohres zumindest bereichsweise mit einer elektrisch leitenden Oberfläche ausgeführt.

Eine besonders robuste Ausführung ist nach der Erfindung dadurch gegeben, dass die Elektroden an der Rohrwand des Messrohres anliegen. Beispielsweise können in der Rohrwand Ausnehmungen für die Elektroden vorgesehen sein, in denen die Elektroden aufgenommen sind.

Insbesondere aus fertigungstechnischen Gründen können die Elektroden jedoch auch von der Rohrwand des Messrohres beabstandet sein. Eine solche Ausführungsform erlaubt auch ein besonders einfaches Auswechseln der Elektroden.

Eine weitere vorteilhafte Ausführungsform der Erfindung besteht darin, dass an der Rohrwand Abstandshalter vorgesehen sind, welche in das Innere des Messrohres vorstehen, und an denen die Elektroden gehaltert sind. Die Abstandshalter können beispielsweise zylindrisch ausgebildet sein. Zweckmäßigerweise verlaufen die Längsachsen der Abstandshalter quer, insbesondere radial, zur Rohrachse.

Weiterhin ist es fertigungstechnisch besonders zweckmäßig, dass zumindest einer der Abstandshalter die Rohrwand durchdringt. In diesem Fall kann der Abstandshalter besonders einfach montiert werden, zum Beispiel durch Befestigungsmittel wie Schraubenmuttern oder andere an der Rohraußenseite. Sofern ein Abstandshalter die Rohrwand durchdringt, ist an diesem zweckmäßigerweise eine Dichtung angeordnet, um ein Austreten von Flüssigkeit aus dem Rohrinneren im Bereich des Abstandshalters zu verhindern. Vorzugsweise können alle Abstandshalter die Rohrwand durchdringen.

Eine weitere bevorzugte Ausführungsform der Erfindung besteht darin, dass zumindest einer der Abstandshalter ein elektrisches Isolatormaterial aufweist. Hierdurch können unerwünschte Nebenschlüsse verhindert werden und die Messgenauigkeit erhöht werden. Bei dem elektrischen Isolatormaterial kann es sich insbesondere um ein Kunststoffmaterial handeln. Es kann ausreichend sein, dass der Abstandshalter das elektrische Isolatormaterial an seiner Oberfläche aufweist. Der Abstandshalter kann aber auch massiv aus dem elektrischen Isolatormaterial gefertigt sein. Vorzugsweise weisen alle Abstandshalter ein elektrisches Isolatormaterial auf.

Eine weitere vorteilhafte Weiterbildung der Erfindung besteht darin, dass zumindest einer der Abstandshalter, z. B. in seinem Inneren, eine Anschlussleitung für einen elektrischen Kontakt mit der gehalterten Elektrode aufweist. In diesem Fall dient der Abstandshalter nicht nur zum Festlegen der Elektrode, sondern auch zum elektrischen Kontaktieren der Elektrode. Zweckmäßigerweise ist jeder Elektrode zumindest ein Abstandshalter zugeordnet, welcher eine solche Anschlussleitung aufweist.

Eine besonders einfache und zugleich zuverlässige Halterung der Elektroden kann dadurch gewährleistet werden, dass zumindest eine der Elektroden an zumindest zwei Halterungsstellen am Messrohr gehaltert ist. Die Halterung erfolgt dabei vorzugsweise über die vorgenannten Abstandshalter. Die Halterungsstellen können insbesondere in zwei gegenüberliegenden Endbereichen der Elektrode vorgesehen sein. Besonders bevorzugt ist es, dass die Halterung der Elektrode an zumindest einer der Halterungsstellen über ein Loslager erfolgt. Das Loslager lässt eine Wärmedehnung und/oder Wärmekontraktion der Elektrode zu. Ein solches Loslager beugt somit thermischen Verspannungen aufgrund unterschiedlicher Ausdehnungskoeffizienten von Elektrode und Messrohrwand vor. In diesem Zusammenhang ist zu beachten, dass im Betrieb Temperaturunterschiede von mehr als 80°C auftreten könne, wobei sich die Temperaturunterschiede insbesondere zwischen der Produkttemperatur und der Temperatur ergeben können, welche bei einem Reinigungsverfahren, insbesondere einem Cleaning in Place (CIP) Verfahren, herrscht. Eine metallische Elektrode kann sich dabei unter Umständen wenig stark ausdehnen als eine isolierende Rohrwand. Das Loslager kann beispielsweise als Bohrung im Abstandshalter ausgebildet sein. An der zweiten Halterungsstelle ist bevorzugt ein Fixlager gegeben, welches beispielsweise durch ein Sackloch im Abstandshalter gebildet sein kann. Zweckmäßigerweise sind beide Elektroden wie beschrieben gehaltert.

Erfindungsgemäß werden die Elektroden für eine Leitfähigkeitsmessung eingesetzt. Demgemäß ist eine Leitfähigkeitsmesseinrichtung vorgesehen, welche zum Messen der Leitfähigkeit zwischen den beiden Elektroden mit den Elektroden in Leitungsverbindung steht. Die Leitfähigkeitsmesseinrichtung kann beispielsweise eine Spannungsquelle und eine Strommesseinrichtung aufweisen.

Im Hinblick auf die Verwendung in einer Annahmeanordnung ist es vorteilhaft, dass eine Pumpe zum Fördern der Flüssigkeit vorgesehen ist. Zweckmäßigerweise ist das Messrohr an der Saugseite der Pumpe an die Pumpe angeschlossen. Auf diese Weise kann die Messanordnung die drei vorgenannten Funktionen Kavitationsmessung, Füllgradmessung und Niveaumessung unmittelbar erfüllen.

Im Hinblick auf die dreifache Funktionalität ist es überdies vorteilhaft, dass das Messrohr leitungsmäßig zwischen einem Speicherbehälter für die Flüssigkeit und der Pumpe angeordnet ist, und/oder dass das Messrohr leitungsgemäß zwischen einem Zulauf für die Flüssigkeit und der Pumpe angeordnet ist. Ein solcher Speicherbehälter kann beispielsweise für die Entgasung der Flüssigkeit vorgesehen sein und an einer Abzweigung der Förderleitung angeordnet sein. Unter einem Zulauf kann eine Zuführöffnung für die Flüssigkeit verstanden werden, die beispielsweise an einem Schlauch und/oder Saugrüssel ausgebildet sein kann.

Die Erfindung betrifft auch die Verwendung der erfindungsgemäßen Messanordnung in einer Flüssigkeitsannahmeanordnung, insbesondere einer Milchannahmeanordnung. Eine solche Annahmeanordnung weist bevorzugt eine Förderleitung auf, die einerseits einen Zulauf für die Flüssigkeit und andererseits eine Verbindung für einen Sammeltank aufweist, und vorzugsweise auch eine Pumpe und/oder eine Durchflussmesseinrichtung.

Erfindungsgemäß kann eine Recheneinrichtung vorgesehen sein, die zur Füllgradkorrektur der Ergebnisse der Durchflussmessung sowohl mit der Durchflussmesseinrichtung als auch mit der erfindungsgemäßen Messanordnung, insbesondere mit deren Elektroden und/oder deren Leitfähigkeitsmesseinrichtung, in Signalverbindung steht.

Es ist erfindungsgemäß, dass die Messanordnung zur Kavitationsmessung, zur Füllgradmessung und zur Niveaubestimmung eingesetzt wird. Dies entspricht der zuvor beschriebenen dreifachen Funktionalität der Messanordnung.

Die Erfindung betrifft auch ein Verfahren zum Betrieb einer Flüssigkeitsannahmeanordnung, insbesondere Milchannahmeanordnung, bei dem mit einer erfindungsgemäßen Messanordnung die Leitfähigkeit der geförderten Flüssigkeit gemessen wird, und die Leistung einer in der Flüssigkeitsannahmeanordnung vorgesehenen Pumpe reduziert wird, wenn mittels der Messanordnung Kavitation nachgewiesen wird. Demgemäß wird der Saugdruck reduziert, wenn Kavitation nachgewiesen wird. Dies erlaubt es, die Pumpenleistung so zu regeln, dass Kavitation gerade noch vermieden wird, so dass ein maximaler Durchsatz ohne Beschädigung der Milch erreicht wird.

Die Erfindung wird nachfolgend anhand bevorzugter Ausführungsbeispiele näher erläutert, welche schematisch in den beiliegenden Figuren dargestellt sind. In den Figuren zeigen:
- Figur 1: eine perspektivische Ansicht einer ersten Ausführungsform einer erfindungsgemäßen Messanordnung;
- Figur 2: eine Längsschnittansicht der Messanordnung aus Figur 1;
- Figur 3: eine Querschnittansicht der Messanordnung aus Figur 1 und 2;
- Figur 4: eine Längsschnittansicht einer erfindungsgemäßen Messanordnung gemäß einer zweiten Ausführungsform;
- Figur 5: die Messanordnung aus Figur 4 in einer um 90 Grad versetzten Längsschnittansicht; und
- Figur 6: eine Flüssigkeitsannahmeanordnung, in welcher eine erfindungsgemä-βe Messanordnung einsetzbar ist.

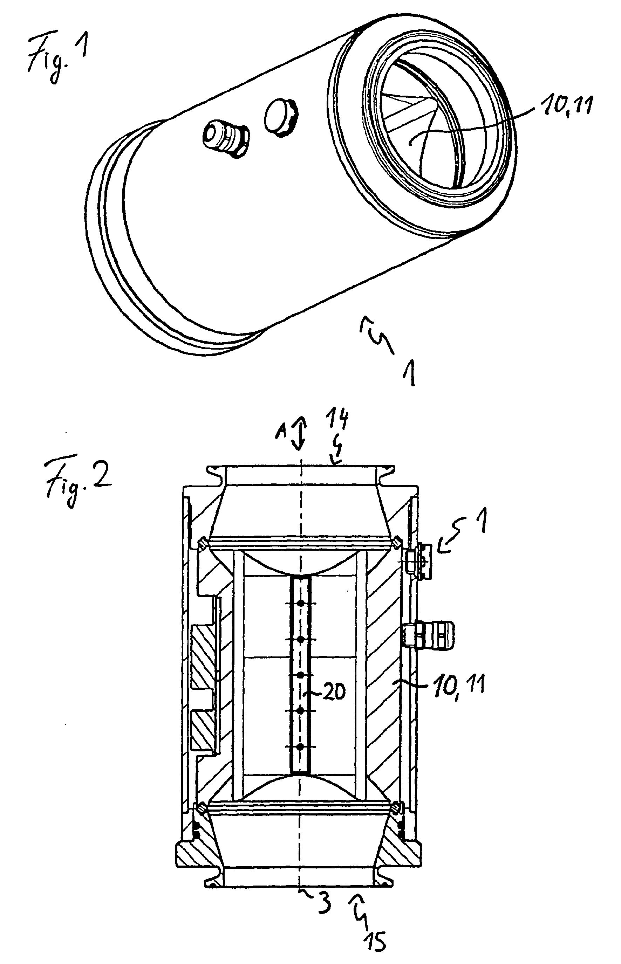

Ein erstes Ausführungsbeispiel einer erfindungsgemäßen Messanordnung 1 ist in Figuren 1 bis 3 dargestellt. Die Messanordnung 1 weist ein Messrohr 10 auf, in dem zwei Elektroden 20, 20' einander diametral gegenüberliegend angeordnet sind. Das Messrohr 10 weist eine Rohrwand 11 auf, die auf Höhe der Elektroden 20 isolierend ausgebildet ist.

Wie Figur 2 zeigt, ist die Elektrode 20 länglich ausgebildet und weist eine rechteckige Kontaktoberfläche mit dem Rohrinneren und mit der zu messenden Flüssigkeit auf. Die Elektrode 20 ist dabei erfindungsgemäß so angeordnet, dass ihre Längsachse parallel zur Rohrachse 3 des Messrohres 10 verläuft, dass also die längliche Elektrode 20 längs der Rohrachse 3 verläuft. Die zweite Elektrode 20' ist analog zur ersten Elektrode 20 ausgeführt.

In Figur 2 ist das Messrohr 10 in seiner Betriebsposition dargestellt, bei der das Messrohr 10 und somit seine Rohrachse 3 senkrecht verlaufen. Bei dieser Konfiguration, bei der auch die Elektroden 20, 20' senkrecht verlaufen, kann das Messrohr 10 erfindungsgemäß in besonders einfacher Weise zur Niveaubestimmung dienen. Im Betrieb kann das Messrohr 10 von der zu vermessenden Flüssigkeit in Pfeilrichtung A durchströmt werden.

Die Rohrwand 11 des Messrohres 10 ist aus einem Isolatormaterial, beispielsweise Kunststoff, ausgeführt. Im Ausführungsbeispiel der Figuren 1 bis 3 liegen die Elektroden 20, 20' dabei unmittelbar an der Rohrwand 11 des Messrohres 10 an.

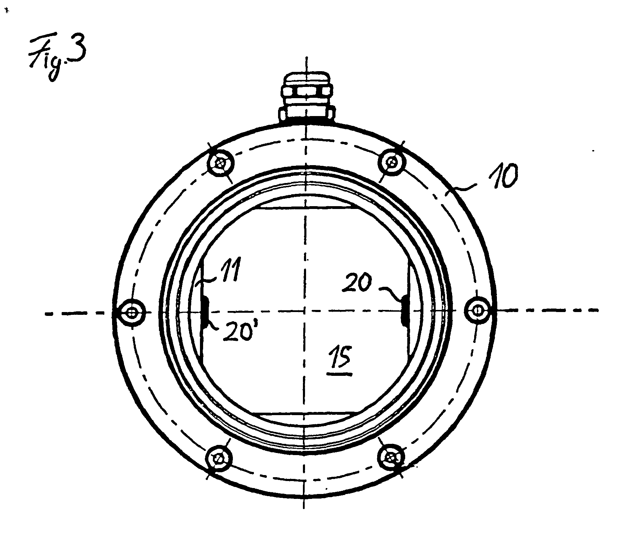

Im Ausführungsbeispiel der Figuren 1 bis 3 weist das Messrohr 10 im Bereich der Elektroden 20 einen rechteckigen, insbesondere quadratischen Rohrquerschnitt auf, wie insbesondere in Figur 3 erkennbar ist. In seinen Endbereichen weist das Messrohr 10 Anschlüsse 14, 15 auf, an denen flanschartige Verbindungseinrichtungen vorgesehen sind. Hier hat das Messrohr für einen besonders strömungsgünstigen Anschluss an übliche Rohrleitungen einen kreisrunden Innenquerschnitt.

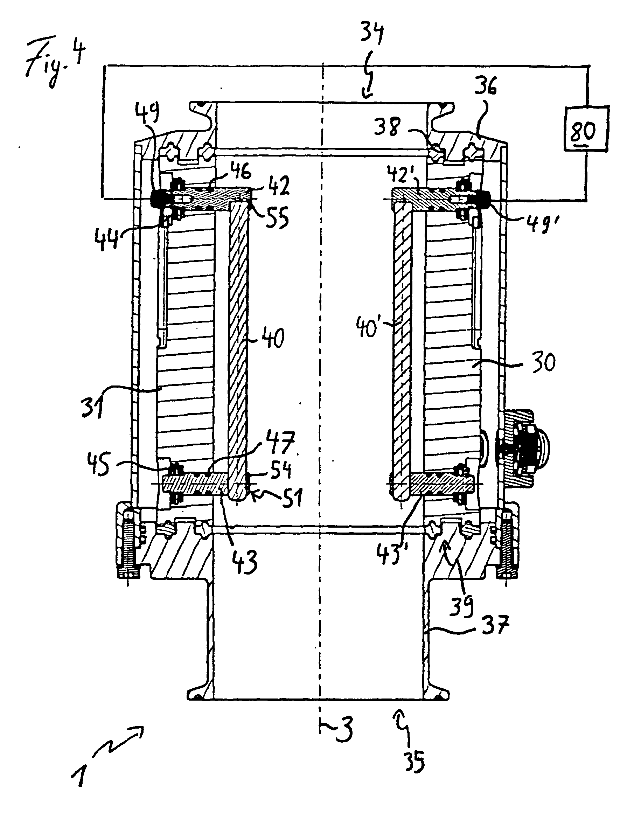

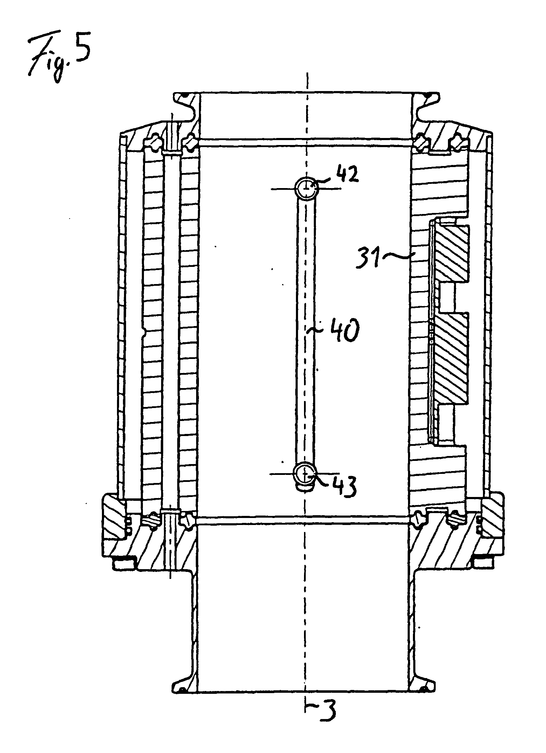

Eine weitere Ausführungsform einer erfindungsgemäßen Messanordnung 1 ist in den Figuren 4 und 5 dargestellt.

Beim Ausführungsbeispiel der Figuren 4 und 5 ist ebenfalls ein Messrohr 30 mit einer isolierenden Rohrwand 31 vorgesehen, an der zwei Elektroden 40 beziehungsweise 40' angeordnet sind. Auch bei der Messanordnung der Figuren 4 und 5 verlaufen die Längsachsen der Elektroden 40, 40' parallel zur Rohrachse 3, wobei die Rohrachse 3 und somit die Elektroden 40, 40' senkrecht verlaufen. Im Gegensatz zum zuvor genannten Ausführungsbeispiel sind beim Ausführungsbeispiel der Figuren 4 und 5 die Elektroden 40, 40' jedoch stabförmig mit beispielsweise rundem, insbesondere kreisrundem Querschnitt, und von der Rohrwand 31 beabstandet ausgeführt. Zum beabstandeten Haltern der Elektroden 40, 40' sind an der Rohrwand 31 Abstandshalter 42, 42', 43, 43' vorgesehen. Von diesen Abstandshaltern 42, 42', 43, 43' werden nachfolgend nur die Abstandshalter 42 und 43 der ersten Elektrode 40 beschrieben. Die Abstandshalter 42', 43' der zweiten Elektrode 40' sind analog aufgebaut.

Die Abstandshalter 42 und 43 sind bolzenförmig ausgebildet und so angeordnet, dass ihre Längsachsen radial zur Rohrachse 3 verlaufen. Die bolzenförmigen Abstandshalter 42, 43 durchdringen die Rohrwand 31 und sind auf der Außenseite der Rohrwand 31 über Schraubmuttern 44 beziehungsweise 45 an der Rohrwand 31 gesichert. Zum Abdichten der Durchgangsöffnungen, welche für die Abstandshalter 42, 43 in der Rohrwand 31 vorgesehen sind, sind an den Abstandshaltern 42, 43 als Dichtringe ausgebildete Dichtungen 46 beziehungsweise 47 vorgesehen, die einerseits an der Rohrwand 31 und andererseits am jeweiligen Abstandshalter 42 beziehungsweise 43 anliegen.

Der obere Abstandshalter 42 weist ein parallel zur Rohrachse 3 verlaufendes Sackloch 55 auf, welches die stabförmige Elektrode 40 aufnimmt und ein axial festes Lager für die Elektrode 40 bildet. Der gegenüberliegende untere Abstandshalter 43 weist eine durchgehende Bohrung 54 auf, welche ein Loslager 51 für die Elektrode 40 bildet. Dieses Loslager 51 erlaubt eine axiale Bewegung der Elektrode 40 relativ zum Abstandshalter 43. Unterschiedliche thermische Ausdehnungen zwischen der metallischen Elektrode 40 und der aus Kunststoff bestehenden Rohrwand 31 führen somit nicht zu einer thermischen Verspannung.

An der Rohraußenseite weist der obere Abstandshalter 42 einen elektrischen Kontakt 49 auf. Dieser Kontakt 49 ist mit einer in den Figuren der Übersichtlichkeit halber nicht dargestellten Anschlussleitung, die im Inneren des Abstandshalters 42 verläuft, mit der Elektrode 40 leitungsverbunden.

Im dargestellten Ausführungsbeispiel sind Abstandshalter 42 und 43 an den Enden der Elektrode 40 angeordnet. Sie können jedoch an beliebigen Orten entlang der Elektrode 40 platziert werden.

Wie in Figur 4 schematisch dargestellt ist, ist überdies eine Leitfähigkeitsmesseinrichtung 80 vorgesehen. Diese Leitfähigkeitsmesseinrichtung 80 dient zur Messung der Leitfähigkeit der Flüssigkeit zwischen den Elektroden 40, 40'. Zu diesem Zweck ist die Leitfähigkeitsmesseinrichtung 80 über die jeweiligen Kontakte 49, 49' mit den Elektroden 40 beziehungsweise 40' verbunden.

Im Ausführungsbeispiel der Figuren 4 und 5 ist das Messrohr 30 sowohl auf Höhe der Elektroden 40, 40' als auch im Bereich der Anschlüsse 34, 35 mit einem kreisrunden Innenquerschnitt ausgebildet. An den beiden gegenüberliegenden Stirnseiten der zylindrischen Rohrwand 31, also im Bereich der Anschlüsse 34, 35, weist das Messrohr 30 Flanschbereiche 36 beziehungsweise 37 auf, die zum Anschluss an ein Rohrleitungssystem dienen. In den Flanschbereichen 36, 37 kann das Messrohr 30 vorzugsweise metallisch ausgebildet sein. Um die beim Betrieb und/oder der Reinigung auftretenden Temperaturunterschiede ausgleichen zu können, insbesondere um unterschiedliche temperaturabhängige Ausdehnungen von Flanschbereichen 36, 37 und Kunststoff-Rohrwand 31 ausgleichen zu können, sind zwischen den Flanschbereichen 36 und 37 einerseits und der Rohrwand 31 andererseits Clampdichtungen 38, 39 angeordnet.

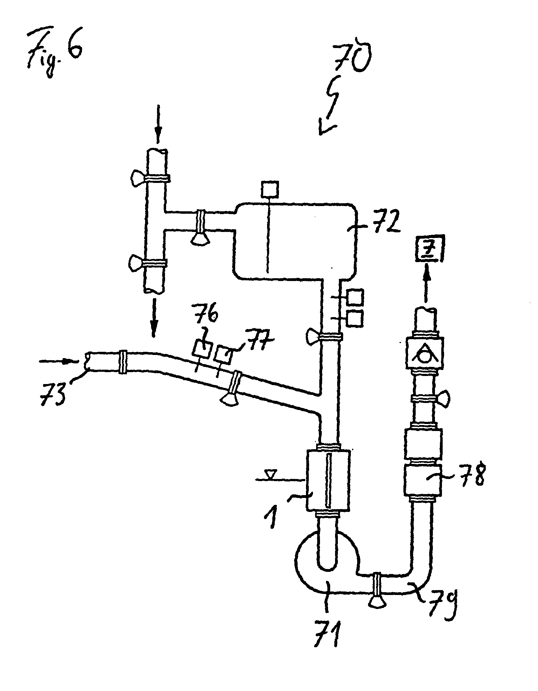

Figur 6 zeigt eine Flüssigkeitsannahmeanordnung, in welcher eine erfindungsgemä-βe Messanordnung 1 zum Einsatz kommen kann.

Die beispielhaft als Milchannahmeanordnung 70 dargestellte Anordnung weist eine Förderleitung 79 auf, die einerseits einen Zulauf 73 für Flüssigkeit, insbesondere Milch aufweist, und die andererseits mit einem lediglich schematisch dargestellten Sammeltank 7 verbunden ist. Im Verlauf der Förderleitung 79 ist eine Pumpe 71 zum Fördern der Flüssigkeit angeordnet. In einem Seitenarm der Förderleitung 79 ist ein Speicherbehälter 72 vorgesehen, der zum Beruhigen der Flüssigkeit beim Annahmebeginn und/oder Annahmeende dienen kann. Im Verlauf der Förderleitung 79 sind überdies ein Milchsensor 76, ein Drucksensor 77 und eine Durchflussmesseinrichtung 78 angeordnet. Der Milchsensor 76 und der Drucksensor 77 sind dabei zwischen dem Zulauf 73 und der Pumpe 71 angeordnet. Die Durchflussmesseinrichtung 78 hingegen ist zwischen der Pumpe 71 und dem Tank 7 angeordnet. Die Messanordnung 1 ist an der Saugseite der Pumpe 71 vorgesehen, das heißt an der Seite der Pumpe 71, welche mit dem Zulauf 73 in Leitungsverbindung steht. Die Messanordnung 1 ist dabei der Pumpe 71 unmittelbar vorgeschaltet.

Die Milchannahmeanordnung 70 mit der erfindungsgemäßen Messanordnung 1 kann wie folgt betrieben werden:

### a) Kavitationsmessung (im Förderbetrieb)

Zu Beginn der Milchannahme wird die Förderleitung 79 und somit die Messanordnung 1 befüllt und der Leitwert der Milch bei stehendem Produkt oder langsamer Fördergeschwindigkeit mittels der Messanordnung 1 ermittelt. Dann wird die Förderleistung, beispielsweise durch Leistungssteigerung der Pumpe 71, erhöht, bis sich der gewünschte Unterdruck einstellt, was mittels des Drucksensors 77 bestimmt werden kann. Der Leitwert wird dabei fortwährend mit der Messanordnung 1 kontrolliert. Soll die Förderleistung bis an die Kavitationsgrenze herangeführt werden, so wird der Saugunterdruck durch Erhöhen der Pumpleistung so lange erhöht, bis der von der Messanordnung 1 gemessene Leitwert abnimmt. Diese Leitwertabnahme ist auf kleinste Gasbläschen zurückzuführen, die sich durch Kavitation bilden. Der Nachweis der Kavitation mittels der Messanordnung 1 ermöglicht es, die Pumpleistung so zu regeln, dass die Pumpe 71 gerade an der Kavitationsgrenze arbeitet.

### b) Füllgradmessfunktion (bei Lufteintrag)

Zum Ende der Annahme hin oder bei Sogbildung kann es zu einem Lufteintrag in der Milch kommen, der nicht auf Kavitation zurückzuführen ist. Auch dieser Lufteintrag wird mittels der Messanordnung 1 gemessen und die Pumpleistung entsprechend reduziert, wodurch unter Umständen der Strudel verkürzt werden kann und somit der Lufteintrag verringert werden kann. Damit der Lufteintrag nicht zu verfälschten Messwerten bei der Durchflussmessung führt, können die Messwerte der Durchflussmesseinrichtung 78 mit den Füllgradmesswerten der Messanordnung 1 korrigiert werden.

Wird der Lufteintrag trotz Reduzierung der Saugleistung der Pumpe 71 größer, so kann in der Regel von einem Abschluss der Annahme ausgegangen werden. Fällt zusätzlich das Signal des Milchsensors 76 ab, so wird die Pumpe 71 gestoppt und der Speicherbehälter 72 wird aktiviert, der die Förderleitung 79 im Bereich des Zulaufs 73 leersaugt.

### c) Niveausensor (am Abschluss der Messung/Übergabe und im Hinblick auf Eichvorgaben)

Zum Ende der Annahme kann die Messanordnung 1 als Niveausensor dienen. Zum Annahmeende wird die Milch aus dem Speicherbehälter 72 gepumpt, wobei das Füllniveau in der Förderleitung 79 aus messtechnischen und eichtechnischen Gesichtspunkten in der Regel auf ein definiertes Abschlussniveau gefahren werden muss, um das Annahmevolumen exakt bestimmen zu können. Denn eine Differenz zwischen Startniveau und Stoppniveau bedingt einen Volumenunterschied, der berücksichtigt werden muss. Auch für diesen Prozessschritt kann die Messanordnung 1 verwendet werden, da sie ein füllniveauabhängiges Signal abgibt und es somit erlaubt, zum Annahmeende ein vorbestimmtes Niveau anzufahren und/oder Niveauunterschiede zwischen Annahmebeginn und Annahmeende nachzuweisen.

## Patentansprüche

1. Messanordnung (1) für eine Flüssigkeit mit
- einem Messrohr (10, 30) zum Durchleiten der Flüssigkeit,
- zwei länglichen Elektroden (20, 40), die zumindest bereichsweise einander
- gegenüberliegend im Messrohr (10, 30) angeordnet sind, und
- einer Leitfähigkettsmesseinrtchiung (80), welche zum Messen der Leitfähigkeit zwischen den beiden Elektroden (20, 40) mit den Elektroden (20, 40) in Leitungsverbindung steht,
wobei
- die länglichen Elektroden (20, 40) längs der Rohrachse (3) des Messrohres (10, 30) verlaufen, und
- die Rohrachse (3) des Messrohres (10, 30) in Betriebsposition im Bereich der Elektroden (20, 40) zumindest annähernd senkrecht verläuft,
**dadurch gekennzeichnet,**
**dass** die Messanordnung zur Kavitationsmessung mit der Flüssigkeit befüllbar ist, wobei das Auftreten von Kavitation durch eine Änderung der Leitfähigkeit zwischen den beiden Elektroden aufgrund von Kavitationsblasen erkannt wird und die Messanordnung in einem Förderbetrieb von der Flüssigkeit, In senkrechter Richtung durchströmt wird.

2. Messanordnung (1) nach Anspruch 1,
**dadurch gekennzeichnet.**
**dass** das Messrohr (10, 30) eine Rohrwand (11, 31) aufweist, die zumindest an der Rohrinnenseite ein elektrisches isolotormaterlal aufweist.

3. Messanordnung (1) nach einem der vorstehenden Ansprüche,
**dadurch gekennzeichnet,**
**dass** die Elektroden (20) an der Rohrwand (11) des Messrohres (10) anliegen.

4. Messanordnung (1) nach einem der Ansprüche 1 oder 2,
**dadurch gekennzeichnet,**
**dass** die Elektroden (40) von der Rohrwand (31) des Messrohres (30) beabstandet sind.

5. Messanordnung (1) nach einem der vorstehenden Ansprüche,
**dadurch gekennzeichnet,**
**dass** an der Rohrwand (31) Abstandshalter (42, 43) vorgesehen sind, welche in das Innere des Messrohres (30) vorstehen, und an denen die Elektroden (40) gehaltert sind.

6. Messanordnung (1) nach Anspruch 5,
**dadurch gekennzeichnet,**
**dass** zumindest einer der Abstandshalter (42, 43) die Rohrwand (31) durchdringt.

7. Messanordnung (1) nach einem der Ansprüche 5 oder 6,
**dadurch gekennzeichnet,**
**dass** zumindest einer der Abstandshalter (42, 43) ein elektrisches isolatormaterial aufweist und
**dass** zumindest einer der Abstandshalter (42) in seinem inneren eine Anschlussleitung für einen elektrischen Kontakt mit der gehalterten Elektrode aufweist

8. Messanordnung (1) nach einem der vorstehenden Ansprüche,
**dadurch gekennzeichnet,**
**dass** zumindest eine der Elektroden (40) an zumindest zwei Halterungsstellen am Messrohr (30) gehaltert ist, wobei die Halterung der Elektrode (40) an zumindest einer der Halterungsstellen über ein Loslager (51) erfolgt.

9. Messanordnung (1) nach einem der vorstehenden Ansprüche,
**dadurch gekennzeichnet,**
**dass** eine Pumpe (71) zum Fördern der Flüssigkeit vorhanden ist, und
**dass** das Messrohr (10, 30) an der Saugseite der Pumpe (71) an die Pumpe (71) angeschlossen ist.

10. Messanordnung (1) nach einem der vorstehenden Ansprüche,
**dadurch gekennzeichnet,**
**dass** das Messrohr (10, 30) leitungsmäßig zwischen einem Speicherbehälter (72) für die Flüssigkeit und der Pumpe (71) angeordnet ist, und/oder
**dass** das Messrohr (10, 30) leitungsmäßig zwischen einem Zulauf (73) für die Flüssigkeit und der Pumpe (71) angeordnet ist.

11. Verwendung der Messanordnung (1) nach einem der vorstehenden Ansprüche in einer Flüssigkeitsannahmeanordnung (70) zur Kavitationsmessung, zur Füllgradmessung und zur Niveaubestimmung.

12. Verfahren zum Betrieb einer Flüssigkeitsannahmeanordnung (70), bei dem mit einer Messanordnung (1)nach einem der Ansprüche 1 bis 10,
die Leitfähigkeit der geförderten Flüssigkeit gemessen wird, und
die Leistung einer in der Flüssigkeitsannahmeanordnung (70) Vorhandenen Pumpe (71) reduziert wird, wenn mittels der Messanordnung (1) Kavitation nachgewiesen wird.

## Claims

1. A measuring arrangement (1) for a liquid having
- a measuring tube (10, 30) through which the liquid is conducted,
- two elongated electrodes (20, 40) which are arranged opposite one another in the measuring tube (10, 30) at least in places, and
- a conductivity measuring device (80), which is in line connection to the electrodes (20, 40) to measure the conductivity between the two electrodes (20, 40), wherein
- the elongated electrodes (20, 40) run along the tube axis (3) of the measuring tube (10, 30) and
- the tube axis (3) of the measuring tube (10, 30) runs at least approximately perpendicularly in the operating position in the region of the electrodes (20, 40),
**characterized in that**
the measuring arrangement can be filled with liquid for cavitation measurement, wherein the occurrence of cavitation is identified by a change in conductivity between the two electrodes on account of cavitation bubbles and the liquid flows through the measuring arrangement in a perpendicular direction in a conveying operation.

2. The measuring arrangement (1) according to claim 1,
**characterized in that**
the measuring tube (10, 30) exhibits a tube wall (11, 31), which exhibits an electrical insulating material at least on the inside of the tube.

3. The measuring arrangement (1) according to one of the preceding claims,
**characterized in that**
the electrodes (20) lie against the tube wall (11) of the measuring tube (10).

4. The measuring arrangement (1) according to one of the claims 1 or 2,
**characterized in that**
the electrodes (40) are spaced apart from the tube wall (31) of the measuring tube (30).

5. The measuring arrangement (1) according to one of the preceding claims,
**characterized in that**
spacers (42, 43) are provided on the tube wall (31), which project into the inside of the measuring tube (30) and on which the electrodes (40) are mounted.

6. The measuring arrangement (1) according to claim 5,
**characterized in that**
at least one of the spacers (42, 43) penetrates the tube wall (31).

7. The measuring arrangement (1) according to one of the claims 5 or 6,
**characterized in that**
at least one of the spacers (42, 43) exhibits an electrical insulating material and
that at least one of the spacers (42) exhibits in its interior a connection line for an electrical contact with the mounted electrode.

8. The measuring arrangement (1) according to one of the preceding claims,
**characterized in that**
at least one of the electrodes (40) is mounted on at least two mounting points on the measuring tube (30), wherein the electrode (40) is mounted on at least one of the mounting points via a floating bearing (51).

9. The measuring arrangement (1) according to one of the preceding claims,
**characterized in that**
a pump (71) for conveying the liquid is present and that the measuring tube (10, 30) is connected to the pump (71) on the suction side of the pump (71).

10. The measuring arrangement (1) according to one of the preceding claims,
**characterized in that**
the measuring tube (10, 30) is arranged with line connection between a storage container (72) for the liquid and the pump (71) and/or that the measuring tube (10, 30) is arranged in line terms between an incoming line (73) for the liquid and the pump (71).

11. A use of the measuring arrangement (1) according to one of the preceding claims in a liquid collection arrangement (70) for cavitation measurement, for filling capacity measurement and for level determination.

12. A method for operating a liquid collection arrangement (70) in which using a measuring arrangement (1) according to one of the claims 1 to 10 the conductivity of the conveyed liquid is measured and the output of a pump (71) present in the liquid collection arrangement (70) is reduced when cavitation is demonstrated by means of the measuring arrangement (1).

## Revendications

1. Dispositif de mesure (1) pour un liquide avec
- un tube de mesure (10, 30) pour faire passer le liquide,
- deux électrodes allongées (20, 40), lesquelles sont disposées au moins sur certaines parties de manière opposée l'une à l'autre dans le tube de mesure (10, 30), et
- un dispositif de mesure de conductivité (80), lequel est en raccordement de conduite avec les électrodes (20, 40) pour la mesure de la conductivité entre les deux électrodes (20, 40), moyennant quoi
- les électrodes allongées (20, 40) s'étendent le long de l'axe de tube (3) du tube de mesure (10, 30) et
- l'axe de tube (3) du tube de mesure (10, 30) s'étendant au moins sensiblement de manière verticale, en position de fonctionnement, dans la zone des électrodes (20, 40),
**caractérisé en ce que** le dispositif de mesure peut être rempli avec le liquide pour la mesure de la cavitation, moyennant quoi l'apparition d'une cavitation est reconnue grâce à une modification de la conductivité entre les deux électrodes en raison de bulles de cavitation et le dispositif de mesure étant traversé par le liquide en direction verticale dans un fonctionnement d'acheminement.

2. Dispositif de mesure (1) selon la revendication 1, **caractérisé en ce que** le tube de mesure (10, 30) présente une paroi de tube (11, 31), laquelle présente un matériau d'isolateur électrique au moins sur le côté intérieur du tube.

3. Dispositif de mesure (1) selon l'une des revendications précédentes, **caractérisé en ce que** les électrodes (20) reposent contre la paroi de tube (11) du tube de mesure (10).

4. Dispositif de mesure (1) selon l'une des revendications 1 ou 2, **caractérisé en ce que** les électrodes (40) sont espacées par rapport à la paroi de tube (31) du tube de mesure (30).

5. Dispositif de mesure (1) selon l'une des revendications précédentes, **caractérisé en ce que** l'on prévoit des éléments espaceurs (42, 43) sur la paroi de tube (31), lesquels dépassent à l'intérieur du tube de mesure (30) et sur lesquels les électrodes (40) sont fixées.

6. Dispositif de mesure (1) selon la revendication 5, **caractérisé en ce qu'**au moins l'un des éléments espaceurs (42, 43) traverse la paroi de tube (31).

7. Dispositif de mesure (1) selon l'une des revendications 5 ou 6, **caractérisé en ce qu'**au moins l'un des éléments espaceurs (42, 43) présente un matériau d'isolateur électrique et
**en ce qu'**au moins l'un des éléments espaceurs (42) présente dans son intérieur une conduite de raccordement pour un contact électrique avec l'électrode fixée.

8. Dispositif de mesure (1) selon l'une des revendications précédentes, **caractérisé en ce qu'**au moins l'une des électrodes (40) est fixée sur le tube de mesure (30) à au moins deux emplacements de fixation, moyennant quoi la fixation de l'électrode (40) à au moins l'un des emplacements de fixation s'effectue via un palier libre (51).

9. Dispositif de mesure (1) selon l'une des revendications précédentes, **caractérisé en ce qu'**il y a une pompe (71) pour l'acheminement du liquide, et **en ce que** le tube de mesure (10, 30) est raccordé à la pompe (71) du côté d'aspiration de la pompe (71).

10. Dispositif de mesure (1) selon l'une des revendications précédentes, **caractérisé en ce que** le tube de mesure (10, 30) est disposé, du point de vue de la conduite, entre un réservoir (72) pour le liquide et la pompe (71), et/ou
**en ce que** le tube de mesure (10, 20) est disposé, du point de vue de la conduite, entre une arrivée (73) pour le liquide et la pompe (71).

11. Utilisation du dispositif de mesure (1) selon l'une des revendications précédentes dans un système collecteur de liquide (70) pour la mesure de la cavitation, pour la mesure du degré de remplissage et pour la détermination du niveau.

12. Procédé pour faire fonctionner un système collecteur de liquide (70), avec lequel on mesure, à l'aide d'un dispositif de mesure (1) selon l'une des revendications 1 à 10, la conductivité du liquide acheminé, et
la puissance d'une pompe (71) se trouvant dans le système collecteur de liquide (70) étant réduite si l'on détermine une cavitation à l'aide du dispositif de mesure (1).
